# FASCICULE DE BREVET EUROPEEN

(11) **EP 2 303 386 B1**
(45) Date de publication et mention de la délivrance du brevet: **18.02.2015**
(21) Numéro de dépôt: 09769467.3
(22) Date de dépôt: 29.06.2009
(51) Int. Cl.: A61M 25/02

(54) **PANSEMENT DE FIXATION ET DE PROTECTION D'AIGUILLE**
VERBAND ZUR FIXIERUNG UND ZUM SCHUTZ EINER NADEL
BANDAGE FOR FIXING AND PROTECTING A NEEDLE

(30) Priorité: 27.06.2008 FR 0803629
(43) Date de publication de la demande: 06.04.2011
(73) Titulaire: Nephrokit, 78110 Le Vesinet (FR)
(72) Inventeur: CHAWKI, Mokhtar, F-78110 Le Vesinet (FR)
(74) Mandataire: Demulsant, Xavier
(86) Numéro de dépôt international: PCT/FR2009/000803
(87) Numéro de publication internationale: WO 2009/156626

(56) Documents cités:
- EP-A- 0 284 219
- US-A- 4 490 141
- US-A- 5 546 938

## Description

L'invention se rapporte au domaine médical ou vétérinaire, et concerne les moyens de maintien d'aiguilles, plus particulièrement, mais non exclusivement, d'aiguilles à ailettes.

L'invention concerne également un dispositif de protection du site d'entrée d'une aiguille. Un tel dispositif est décrit dans le document EP 284219.

L'invention intéresse notamment l'utilisation médicale ou vétérinaire des cathéters intraveineux.

L'invention trouve notamment une application dans le domaine de l'hémodialyse, ou celui des perfusions à aiguilles/cathéters ou dispositifs intraveineux courts de type cathlon ® (canules en PTFE).

L'invention concerne entre autres un dispositif de fixation d'aiguilles à ailettes au site de ponction veineuse lors d'une perfusion veineuse ou artérioveineuse, en particulier pour fistule artérioveineuse chez l'hémodialysé.

Il est courant de retirer ou d'injecter des fluides (électrolytes, sérum glucosé isotonique anesthésique...), ou d'administrer des médicaments à un patient ou un animal par l'intermédiaire d'un tube fixé à une aiguille ou un cathéter.

Cette aiguille ou ce cathéter doivent être insérés manuellement et en un emplacement précis, par exemple en intraveineuse, en intramusculaire, en sous-cutané.

Les aiguilles à ailettes sont employées pour les transfusions, les prises de sang notamment. Ces aiguilles tirent leur nom de ce qu'elles sont pourvues d'une zone de pincement manuel en forme d'ailes de papillon. Le repli de ces ailettes l'une contre l'autre permet une bonne préhension et facilite l'insertion de l'aiguille sous la peau. Une fois l'aiguille mise en place, les ailettes viennent classiquement se reposer sur la peau et une bande adhésive est placée sur ces ailettes et sur la peau en vue d'éviter l'extraction de l'aiguille.

Des exemples d'aiguilles à ailettes peuvent être trouvés dans les brevets américains délivrés sous les numéros suivants: 2 725 058, 3 064 648, 3 640 275, 4 194 504, 4 300 553, 4 627 842, 5 108 376, 5 149 328, 6 270 480.

Lorsque l'aiguille est insérée dans un vaisseau sanguin, même un faible mouvement de cette aiguille peut entraîner un risque de phlébite ou d'hématome, l'aiguille traversant le vaisseau de part en part.

Conventionnellement, les aiguilles sont maintenues en place par l'emploi d'une grande quantité de morceaux de bandes adhésive. La qualité du maintien de l'aiguille dépend donc notamment du soin de la personne qui met ces bandes adhésives en place. Ces bandes adhésives sont inconfortables et peuvent entraîner des problèmes de peau.

L'emploi de bandes adhésives conventionnelles ne permet pas d'éviter les risques d'extraction accidentelle des aiguilles, par exemple lors de perfusions intraveineuses réalisées pour des enfants, qui acceptent mal de rester immobiles durant de longues périodes.

Les mouvements incontrôlés de personnes souffrant de maladies neurodégénératives (Parkinson, Alzheimer) peuvent également entraîner un arrachement des aiguilles, avec perte de produits essentiels au maintien de la vie du patient ou de son équilibre physique ou mental, par exemple sang, produits curatifs, éléments nutritifs, produits de soins palliatifs.

L'emploi des bandes adhésives conventionnelles masque le plus souvent au moins en partie le tubage, de sorte qu'il est parfois difficile de contrôler visuellement le bon déroulement de l'injection ou du drainage des fluides.

Le retrait périodique des bandes adhésives pour effectuer ce contrôle est fastidieux, désagréable pour le patient, et multiplie les risques de mouvement accidentel de l'aiguille.

Il est fréquent que ces bandes adhésives collent aux gants de l'opérateur, avec les risques de piqûre connus et redoutés des infirmiers. L'expansion du SIDA et des hépatites, entre autres maladies contagieuses par voie sanguine, n'a fait que rendre ce risque plus redouté. Or, les adhésifs conventionnels adhèrent aux ailettes des aiguilles et collent aux doigts de gants, provoquant une traction de l'aiguille dont le biseau peut accidentellement piquer l'opérateur.

Le retrait rapide de l'aiguille est parfois souhaité et n'est pas facile lorsque cette aiguille est fixée par une multitude de bandes adhésives posées à la diable.

Lorsque la peau du patient est humide, par suite d'un écoulement de fluide ou de la transpiration, certaines des bandes adhésives peuvent se décoller de la peau, de sorte que l'aiguille n'est plus correctement fixée.

Il est estimé qu'environ 80 % des patients hospitalisés font l'objet d'un traitement administré par cathéter intraveineux. Bien que les cathéters intraveineux périphériques soient moins sujets à infection que les cathéters intraveineux profonds et cathéters veineux centraux, les infections à staphylocoques de cathéters veineux périphériques ne sont pas exceptionnelles. Les mouvements d'aiguilles sont présumés favoriser ces infections.

Pour certains patients, les cathéters périphériques sont d'usage chronique. C'est le cas notamment des malades soufrant d'insuffisance rénale aiguë ou chronique et traités par hémodialyse, ou épuration extra rénale.

La séance d'hémodialyse dure environ quatre heures, et doit être effectuée trois fois par semaine.

Trois types d'accès vasculaires prédominent pour l'hémodialyse: les fistules artérioveineuses FAV, les prothèses artérioveineuses ou grafts, et les cathéters veineux centraux CVC.

Les FAV sont des anastomoses créées chirurgicalement pour connecter une artère et une veine du patient, couramment dans l'avant bras, ou le bras, le plus souvent entre une artère radiale ou humérale et sa veine homonyme. Cette anastomose permet d'augmenter le débit sanguin au sein de ladite veine. La création d'une FAV modifie fortement l'aspect de l'avant bras du patient, par création de zones anévrismales. C'est la raison pour laquelle de nombreux dispositifs rigides de maintien d'aiguilles, connus dans l'art antérieur, ne peuvent être utilisés pour le maintien des aiguilles de dialyse sur une FAV.

Les aiguilles utilisées pour ponctionner les FAV sont de gros calibre, d'un diamètre interne variant typiquement entre 1.6 et 2 mm. L'aiguille amenant le sang du patient vers l'appareil de dialyse est appelée artère, celle restituant le sang vers le patient étant appelée veine.

Lors d'une séance d'hémodialyse d'un patient, plusieurs incidents ou accidents doivent être évités.

Une mauvaise fixation de l'aiguille représente un réel danger pour le patient, étant donné les débits élevés de fonctionnement d'une FAV. Toute hémorragie à partir du point de ponction peut s'avérer fatale pour le patient. De plus, lors de l'hémodialyse, un anticoagulant est employé pour limiter les risques de colmatage des lumières des cathéters par thrombose. Du fait des débits de sang élevés et de l'utilisation d'anticoagulant, les risques liés à un arrachement accidentel des aiguilles de dialyse sont très importants.

Il est fréquent d'observer un saignement local entre la paroi cutanée et le point d'insertion de l'aiguille, ceci étant un facteur de contamination infectieuse à travers la paroi de ponction. Pour éviter cet écueil, les dispositifs de fixation doivent imposer à l'aiguille une pression de pénétration permanente pendant la dialyse.

Pendant la ponction ou au cours d'une séance de dialyse, une aiguille peut être transfixiante. En d'autres termes, l'aiguille peut traverser les parois du vaisseau et induire un hématome. Lorsqu'il s'agit de l'aiguille veineuse, le sang est réinjecté sous pression pendant la séance de dialyse et crée un volumineux hématome. Plus rarement, l'aguille pénètre l'artère sous-jacente, ce qui provoque un hématome profond. Un anévrysme peut se développer sur l'artère, plus tardivement. Un tel anévrysme nécessite un traitement chirurgical. Pendant la dialyse, un hématome peut survenir lorsque l'aiguille est fixée après avoir embroché partiellement la paroi du vaisseau. Cette aiguille peut devenir transfixiante lors d'un mouvement désordonné ou involontaire du patient. Il faut alors arrêter prématurément la séance de dialyse, au prix d'une mauvaise qualité de l'épuration extra rénale.

La sortie accidentelle d'une aiguille alors que la circulation extra corporelle est fonctionnelle est un accident grave. C'est en particulier très grave lorsqu'il s'agit de l'aiguille veineuse. Un tel accident expose au décès du patient par hémorragie iatrogène. En l'absence d'alarme spécifique sur la circulation extra corporelle, la pompe à sang continue de fonctionner et vide le patient de son sang.

Un tel accident est susceptible de se produire par exemple chez les patients agités, ou dépressifs, épileptiques, ou atteints de chorée, d'une maladie neurodégénérative telle que Parkinson, syndrome parkinsonien, ou frappés de convulsions pour diverses raisons.

Le débranchement inopiné de l'aiguille artérielle expose au saignement au point de ponction et au risque d'embolie gazeuse. Bien que rare grâce à la présence de détecteur d'air dans les appareils de dialyse moderne, ce risque reste réel lorsque les débits sanguins sont élevés, en raison de la latence de réponse du clamp de sécurité.

Le débranchement simultané des deux aiguilles aura pour le malade deux conséquences: une spoliation de 250 cc de sang, un saignement aux points de ponction. Ce saignement nécessitera l'interruption de la séance de dialyse et une compression avant démarrage éventuel d'une nouvelle séance.

Les prothèses ou graft sont mises en place par voie chirurgicale, et conduisent également à un lien artérioveineux, assuré par un conduit en matériau polymère biocompatible. Les aiguilles de dialyse doivent être insérées dans ce conduit synthétique, ce qui nécessite un effort manuel assez important.

Les risques liés au débranchement des aiguilles de dialyse implantées dans une FAV sont sensiblement identiques à ceux d'un débranchement des aiguilles de dialyse implantées dans un graft.

Les aiguilles à ailettes pour hémodialyse sont traditionnellement fixées avec plusieurs bandes adhésives, dont au moins une est collée en chevron ou en cravate, en faisant passer l'adhésif en dessous de la tubulure, puis au-dessus des ailettes.

Les dispositifs de fixation conventionnels des cathéters ou aiguilles de perfusion ne peuvent être employés chez la plupart des dialysés et ce pour les raisons suivantes.

En premier lieu, un certain nombre de ces dispositifs antérieurs sont rigides. Or, la FAV, abord vasculaire de première indication et le plus répandu, entraîne l'apparition de zones anévrismales, la peau étant très déformée au voisinage de la FAV. L'utilisation de dispositifs de fixation rigides provoque une irritation répétée de la peau, favorisant les excoriations particulièrement pourvoyeuses de contamination bactérienne. De plus, les fistules peuvent être maintenues actives pendant de très nombreuses années, et la peau de certains dialysés est peu souple, mince et fragile du fait de l'âge.

En second lieu, un certain nombre de dispositifs antérieurs de fixation d'aiguilles comportent des sangles. De telles sangles sont tout à fait proscrites pour les patients porteurs d'une FAV, du fait du risque de garrottage pouvant entraîner une ischémie.

En troisième lieu, un grand nombre de ces dispositifs antérieurs de fixation d'aiguille sont de grande taille. Or, les FAV sont souvent de petite longueur, de sorte que les deux aiguilles artère et veine doivent être placées proches l'une de l'autre.

Le document EP 0284219 décrit un pansement de fixation sur la peau d'un cathéter ou d'une canule. Le pansement est pourvu d'une ligne séparatrice longitudinale prolongée par un trou oblong pour le passage d'un connecteur de canule.

Le pansement décrit dans le document EP 0284219 présente de nombreux inconvénients. Sa fabrication est complexe et onéreuse. En particulier, ce pansement de l'art antérieur comporte une première bande pelable, dont l'extrémité forme patte de préhension, cette première bande pelable devant être placée repliée sur elle-même, entre une masse adhésive et la partie extrême d'une deuxième bande pelable. La pose et l'enlèvement de ce pansement de l'art antérieur est délicate. Lors de la pose, l'infirmier doit en effet saisir à deux mains le pansement pour enlever les deux bandes pelables, dégageant ainsi la surface inférieure du pansement, entièrement adhésive. Les risques de collage intempestifs sur les gants de l'infirmier sont élevés lors de la pose ou de l'enlèvement du pansement.

Le document US 4490141 décrit un dispositif de fixation de cathéter. Le dispositif décrit dans ce document antérieur présente de nombreux inconvénients. En particulier, ce dispositif ne recouvre pas le site de ponction et ne le protège pas des éventuelles contaminations bactériennes. Par ailleurs, ce dispositif antérieur ne permet pas de limiter efficacement les risques de déplacement de l'aiguille.

Après une étude approfondie des problèmes ci-dessus présentés, le présent inventeur a imaginé un produit apportant de nombreux avantages permettant d'atténuer largement les difficultés mentionnées.

En totale opposition avec les moyens conventionnels de l'art antérieur, notamment ceux décrits dans les documents de brevets US 4 863 432, US 4 534 762, US 4 490 141, US 5 087 248, le présent inventeur propose un dispositif dans lequel l'aiguille, notamment une aiguille à ailettes, n'est sensiblement pas collée sur la peau, et n'est pas non plus en contact avec une quelconque bande adhésive, mais reste, en dépit de ces choix a priori contre nature, fermement en position.

L'invention se rapporte, selon un premier objet, à un dispositif de fixation et de protection d'une aiguille, ce dispositif comprenant une première et une deuxième partie adhésives, séparées par une partie centrale, la première partie étant destinée à être fixée sur la peau, avantageusement en regard du site de pénétration choisi pour l'aiguille, seule une des faces de la première partie étant adhésive, ce dispositif comprenant une ligne de découpe définissant deux bras longitudinaux, chacun de ces deux bras comprenant une partie extrême appartenant à la deuxième partie adhésive.

Avantageusement, la première partie est adhésive sur sa face avant, la deuxième partie étant adhésive sur sa face arrière uniquement.

Avantageusement, le pansement est réalisé en un matériau élastique, en particulier suivant les directions longitudinale et transversale. Le pansement peut ainsi suivre les courbures de son support, par exemple les courbures du corps.

Avantageusement, le pansement réalisé en matériau transparent, translucide ou semi transparent, au moins pour sa partie centrale. Une détection visuelle d'anomalie, notamment au point de pénétration, est ainsi facilitée.

Dans une mise en oeuvre, le pansement comprend une fente, notamment longitudinale, s'étendant dans sa partie centrale. Cette fente s'étend au moins en partie dans la première partie adhésive.

Avantageusement, la deuxième partie est de forme sensiblement identique à la première partie, par exemple ovale, carrée ou rectangulaire. La fabrication du pansement est ainsi rendue économique.

L'invention se rapporte, selon un deuxième aspect, à un kit de perfusion comprenant un dispositif tel que présenté ci-dessus et comprenant au moins une aiguille ou moyen analogue.

D'autres objets et avantages de l'invention apparaîtront au cours de la description suivante de modes de réalisation actuellement préférés, description qui va être effectuée en se référant aux dessins annexés, dans lesquels:
- la figure 1 est une vue en plan d'un pansement de fixation et de protection d'aiguille;
- les figures 2 à 7 sont des vues schématiques en perspective illustrant la mise en place d'un pansement tel que représenté en figure 1, pour une aiguille introduite, par exemple, dans l'avant bras d'un patient ;
- la figure 8 est une vue en plan d'un pansement de fixation et de protection d'aiguille, selon une variante de réalisation.

Le pansement de fixation et de protection d'aiguille 1 représenté sur les figures annexées sera, afin de simplicité, désigné par le terme «pansement» dans la description ci-dessous.

Dans la suite de cette description, le terme « longitudinal » est employé en référence à une première direction d'élancement du pansement et le terme « transversal » à une direction sensiblement perpendiculaire.

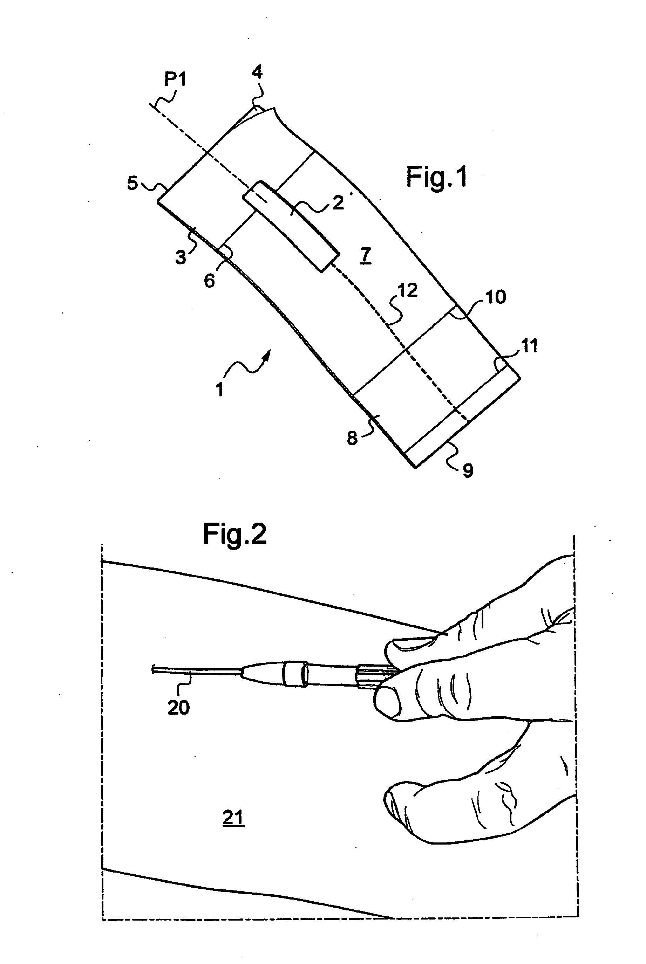

L'on se reporte tout d'abord à la figure 1.

Le pansement 1 se présente sous une forme générale de bande élancée.

Dans le mode de réalisation représenté, cette bande élancée est sensiblement rectangulaire et le pansement est pourvu d'un plan de symétrie longitudinal P1.

Le pansement 1 comprend une fente 2, longitudinale. Ainsi qu'il apparaîtra dan la suite de cette description, les dimensions de cette fente longitudinale 2, et notamment sa dimension transversale, sont adaptées aux dimensions du dispositif de ponction, en particulier au diamètre d'un embout supportant une aiguille.

Dans le mode de réalisation représenté, cette fente 2 est de forme rectangulaire. Dans d'autres modes de réalisation, non représentés, cette fente est ovale, circulaire, carrée ou polygonale.

Dans le mode de réalisation représenté, cette fente 2 est située à mi largeur du pansement. Dans d'autres modes de réalisation, non représentés, cette fente est décentrée et plus près d'un bord longitudinal du pansement.

Sur une première partie 3, dite avant, le pansement 1 est adhésif sur sa première face, dite inférieure.

Une bande pelable 4 protège cette face avant inférieure adhésive, préalablement à la pose du pansement. Cette bande pelable 4 comporte avantageusement une patte de préhension. Dans une réalisation, cette patte de préhension s'étend au-delà du bord extrême libre transversal 5 du pansement 1.

Dans le mode de réalisation représenté, la face avant inférieure adhésive s'étend transversalement au pansement et définit une surface adhésive sensiblement rectangulaire s'étendant entre le bord extrême libre transversal 5 du pansement 1 et un premier bord transversal 6 d'une zone centrale 7 du pansement 1. Dans une mise en oeuvre, la patte de préhension de la bande pelable recouvre légèrement la zone centrale 7, au-delà du premier bord transversal 6.

Dans le mode de réalisation représenté, la fente longitudinale 2 s'étend principalement dans la zone centrale 7 du pansement, et s'étend légèrement au-delà du premier bord transversal 6 de la zone centrale 7. Dans d'autres modes de réalisation, non représentés, la fente longitudinale 2 s'étend jusqu'au voisinage immédiat du premier bord libre transversal 6 mais ne s'étend pas dans la première partie 3.

Sur une deuxième partie 8, dite arrière, le pansement 1 est adhésif sur sa deuxième face dite supérieure.

Une bande pelable protège cette face arrière supérieure adhésive, préalablement à la pose du pansement. Avantageusement, cette bande pelable est pourvue d'une patte de préhension. Dans une mise en oeuvre, cette patte de préhension recouvre légèrement la partie centrale 7.

Dans le mode de réalisation représenté, la face arrière supérieure adhésive s'étend transversalement au pansement et définit une surface adhésive sensiblement rectangulaire s'étendant entre un bord extrême libre transversal 9 du pansement et un deuxième bord transversal 10 de la zone centrale 7 du pansement.

Grâce à des lignes de décollement 11, le retrait des bandes ou films pelables protégeant les surfaces adhésives peut être effectué suivant des plans précis. La fixation du pansement est ainsi facilitée, sans risque de collage aux gants de l'opérateur, ni de froncement.

Le pansement 1 est pourvu d'une ligne de découpe longitudinale 12.

Dans le mode de réalisation représenté, cette ligne de découpe part du bord transversal 9 et va jusqu'à la fente longitudinale 2.

Dans d'autres modes de réalisation, non représentés, cette ligne de découpe ne va pas jusqu'à la fente 2. Une telle mise en oeuvre est utile lorsque la tubulure de perfusion est cylindrique et très souple, avec risque d'écrasement.

Par ligne de découpe, on désigne ici toute ligne de rainage, rainurage, perforations, prédécoupe, amincissement, la technique mise en oeuvre pour réaliser cette ligne d'affaiblissement étant fonction, ainsi qu'il est connu en soit, du type de matériau employé pour le pansement.

Par ligne de découpe, on désigne également toute trace sur le pansement guidant l'opérateur pour la découpe du pansement à l'aide d'un outil.

Dans un mode de mise en oeuvre avantageux, le pansement est réalisé en matériau polymère souple ou tissu enduit, la ligne d'affaiblissement étant une ligne de prédécoupe. Par « souple » on désigne ici une capacité à épouser les courbures d'une surface de pose du pansement, par exemple un avant bras d'un patient, cette souplesse étant assurée par le choix du matériau et de son épaisseur. Par « souple » on désigne ici également une capacité, pour le matériau du pansement, à supporter un certain étirement.

Le pansement est avantageusement transparent, translucide ou semi transparent, en particulier dans sa zone centrale 7.

A titre d'exemple, les dimensions du pansement représenté en figure 1 sont les suivantes :
a) pour la première partie 3 dite partie avant, dimensions de la surface adhésive : 40mm par 25mm ;
b) pour la deuxième partie 8 dite partie arrière, dimensions de la surface adhésive : 40mm par 15 mm, distance entre le bord libre transversal 9 et la ligne de décollement : 5 mm ;
c) pour la partie centrale 7, distance entres les bords transversaux 6,10 : 60mm, largeur de la partie centrale 40mm ;
d) pour la fente longitudinale 2 : largeur 7mm, longueur 27mm ;
e) pour les bandes pelables : largeurs des pattes de décollement 4mm.

On se reporte maintenant aux figures 2 et suivantes qui illustrent une mise en place du pansement.

Dans une première étape, représentée en figure 2, une aiguille 20, par exemple une aiguille de perfusion, est mise en place, par exemple dans l'avant bras 21 d'un patient. Cette introduction de l'aiguille est conventionnelle. Cette aiguille peut être celle d'un cathlon ®, ou bien encore une aiguille à ailettes. L'aiguille est par exemple introduite dans un vaisseau sanguin, ou dans une FAV.

Puis, l'opérateur enlève la bande pelable 4 recouvrant la face inférieure avant adhésive du pansement.

L'opérateur vient fixer cette face inférieure avant contre la peau du patient.

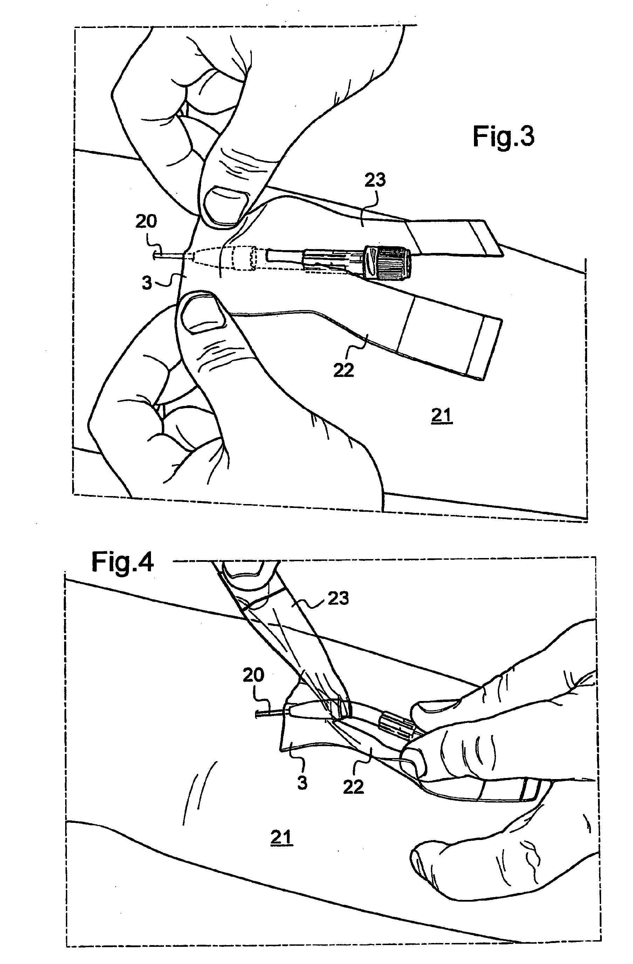

Ainsi qu'il est représenté en figure 3, la face inférieure avant adhésive du pansement 1 vient alors se placer de part et d'autre de l'aiguille et recouvre cette aiguille sur une faible distance, de l'ordre de quelques millimètres.

L'opérateur a séparé ou sépare les deux bras 22, 23 du pansement 1, si nécessaire en découpant ce pansement le long de la ligne d'affaiblissement 12.

La fente longitudinale 2 permet d'écarter les deux bras 22, 23 du pansement 1, en tenant compte du volume de l'extrémité de la tubulure.

L'opérateur saisit un premier bras 23 du pansement, situé à droite de l'aiguille, et fait passer ce bras sous la tubulure, pour le replier ensuite sur la tubulure (figure 4). La bande pelable protégeant la face supérieure adhésive du pansement étant encore en place, le bras 23 ne colle pas aux doigts de l'opérateur.

L'opérateur fixe le premier bras 23 du pansement sur la peau du patient.

Puis l'opérateur saisit le deuxième bras 22 du pansement, situé à gauche de l'aiguille, et fait passer ce bras sous la tubulure, pour le replier ensuite sur la tubulure (figure 5). La bande pelable protégeant la face supérieure adhésive du pansement étant encore en place, le bras 22 ne colle pas aux doigts de l'opérateur.

L'opérateur fixe le deuxième bras 22 du pansement sur la peau du patient.

Il est à noter que l'opérateur peut utiliser une aiguille à ailettes, en repliant les ailettes pour mieux saisir et diriger l'aiguille, et en relâchant les ailettes lorsque l'aiguille est mise en place, les ailettes venant alors reposer contre la face avant du pansement 1, de part et d'autre de la fente 2. La largeur de la fente 2 est alors sensiblement inférieure à la largeur des ailettes lorsque ces ailettes sont en position relâchée.

Le cas échéant, des bandes de renfort longitudinales sont placées de part et d'autre de la fente 2.

L'invention ne nécessite pas pour l'opérateur de changer ses habitudes pour ce qui est de l'introduction de l'aiguille au travers de la peau, ce qui est très sécurisant pour lui.

La souplesse du pansement permet de suivre les contours de la peau, y compris au voisinage des zones anévrismales des FAV. Avantageusement, le matériau constituant les bras 22, 23 et le cas échéant tout le pansement 1, est extensible suivant au moins une direction longitudinale et, encore plus avantageusement, suivant les deux directions longitudinale et transversale. Le pansement est ainsi encore plus adaptable aux différentes courbures du corps du patient.

Il est à noter que l'aiguille, notamment les ailettes d'une aiguille à ailettes ne sont plus collées sur la peau, et ne sont pas non plus en contact avec une zone adhésive. Seule la partie extrême des bras 22, 23 est adhésive. L'enlèvement de l'aiguille est ainsi rendu plus sûr que dans les dispositifs antérieurs. Il suffit en effet de décoller les bras 22, 23, collés à distance de l'aiguille, puis de décoller la partie avant du pansement, cette partie avant ne recouvrant l'aiguille que sur une faible surface.

Le pansement permet de visualiser le site de ponction et de déceler une anomalie éventuelle.

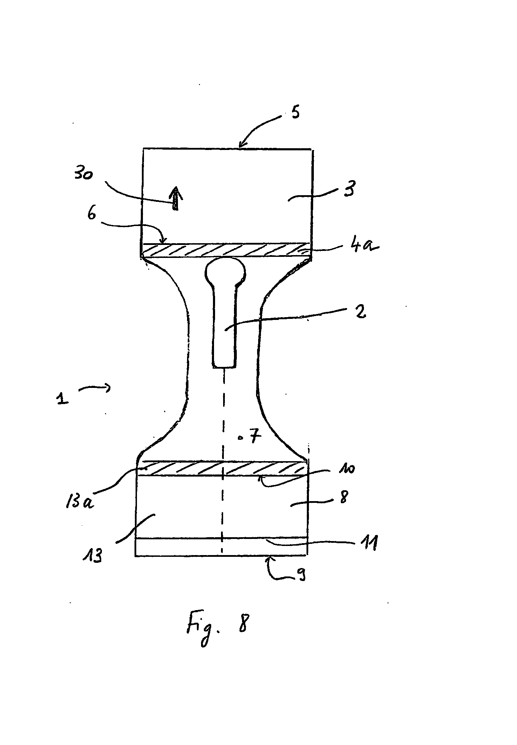

L'on se reporte maintenant à la figure 8 qui illustre différentes dispositions particulières de mises en oeuvre.

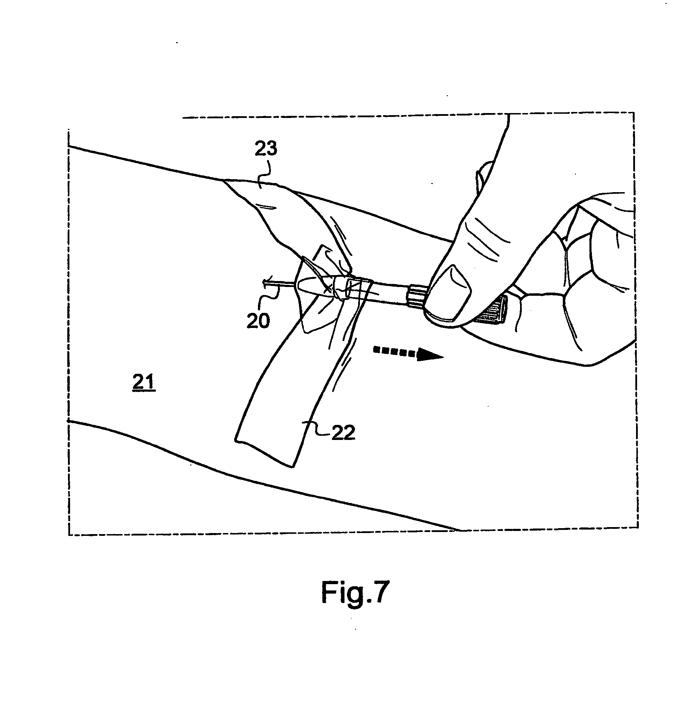

Afin de simplification, l'ensemble de ces dispositions est intégré dans une seule figure. Il est entendu cependant que chacune de ces dispositions peut être utilisée dans un pansement du type décrit précédemment en référence aux figures 1 à 7.

Selon une première disposition particulière, la fente longitudinale 2 présente avantageusement une forme en trou de serrure, cette disposition facilitant le maintien de l'embout de l'aiguille.

Selon une deuxième disposition particulière, un indicateur, par exemple une flèche 30 indique le sens de pose du pansement.

Selon une troisième disposition particulière, la partie centrale 7 est de faible largeur, cette disposition facilitant la mise en place des bras lors de la pose du pansement.

Selon une quatrième disposition particulière, les pattes de préhension 4a, 13a des bandes pelables 4, 13 sont pourvues d'un marquage, par exemple colorées pour faciliter leur position et utilisation.

Selon une cinquième disposition particulière, un médicament est mélangé à la mase adhésive.

Selon une autre disposition particulière, le matériau formant le pansement est pourvu de microperforations.

## Revendications

1. Dispositif de fixation et de protection d'une aiguille, ce dispositif comprenant une première et une deuxième partie adhésives (3,8), séparées par une partie centrale (7), la première partie (3) étant destinée à être fixée sur la peau, en regard du site de pénétration choisi pour l'aiguille, seule une des faces de la première partie (3) étant adhésive, ce dispositif étant **caractérisé en ce qu'**il comprend une ligne de découpe (12) définissant deux bras longitudinaux (22,23), chacun de ces deux bras (22,23) comprenant une partie extrême appartenant à la deuxième partie adhésive (8).

2. Dispositif selon la revendication 1, **caractérisé en ce que** la première partie (3) est adhésive sur sa face avant, la deuxième partie (8) étant adhésive sur sa face arrière uniquement.

3. Dispositif selon la revendication 1 ou 2, **caractérisé en ce qu'**il est réalisé en un matériau élastique, en particulier suivant les directions longitudinale et transversale.

4. Dispositif selon l'une quelconque des revendications 1 à 3, **caractérisé en ce qu'**il est réalisé en matériau transparent, translucide ou semi transparent, au moins pour sa partie centrale.

5. Dispositif selon l'une quelconque des revendications précédentes, **caractérisé en ce qu'**il comprend une fente (2) s'étendant dans sa partie centrale (7).

6. Dispositif selon la revendication 5, **caractérisé en ce que** la fente est longitudinale (2) et s'étend au moins en partie dans la première partie (3) adhésive.

7. Dispositif selon l'une quelconque es revendications précédentes, **caractérisé en ce que** la deuxième partie (8) est de forme sensiblement identique à la première partie (3), par exemple ovale, carrée ou rectangulaire.

8. Kit de perfusion comprenant un dispositif tel que présenté dans l'une quelconque des revendications précédentes et une aiguille (20).

## Patentansprüche

1. Vorrichtung zum Befestigen und Schützen einer Nadel, wobei diese Vorrichtung einen ersten und einen zweiten Klebeteil (3, 8), die durch einen mittleren Teil (7) getrennt sind, umfasst, wobei der erste Teil (3) dazu gedacht ist, auf der Haut gegenüber der Durchtrittsstelle für die Nadel befestigt zu werden, wobei nur eine der Seiten des ersten Teiles (3) klebend ist, wobei diese Vorrichtung dadurch gekennzeichet ist, dass sie eine Schnittlinie (12) umfasst, die zwei Längsarme (22, 23) definiert, wobei jeder dieser beiden Arme (22, 23) einen Endteil umfasst, der zu dem zweiten Klebeteil (8) gehört.

2. Vorrichtung nach Anspruch 1, **dadurch gekennzeichnet, dass** der erste Teil (3) auf seiner Vorderseite klebend ist, wobei der zweite Teil (8) nur auf seiner Rückseite klebend ist.

3. Vorrichtung nach Anspruch 1 oder 2, **dadurch gekennzeichnet, dass** sie aus einem insbesondere in den Längs- und Querrichtungen elastischen Material ausgebildet ist.

4. Vorrichtung nach einem der Anspruche 1 bis 3, **dadurch gekennzeichnet, dass** sie mindestens für ihren mittleren Teil aus einem durchsichtigen, durchscheinenden oder halb durchsichtigen Material ausgebindet ist.

5. Vorrichtung nach einem der vorhergehenden Ansprüche, **dadurch gekennzeichnet, dass** sie einen Schlitz (2) umfasst, der sich in ihrem mittleren Teil (7) erstreckt.

6. Vorrichtung nach Anspruch 5, **dadurch gekennzeichnet, dass** der Schlitz längsgerichtet (2) ist und sich mindestens teilweise in dem ersten Klebeteil (3) erstreckt.

7. Vorrichtung nach einem der vorhergehenden Absprüche, **dadurch gekennzeichnet, dass** der zweite Teil (8) im Wesentlichen die gleiche, beilspielsweise ovale, quadratische oder recheckige Form wie der erste Teil (3) aufweist.

8. Infusionsset, umfassend eine Vorrichtung wie in einem der vorhergehenden Ansprüche vorgelegt und eine Nadel (20).

## Claims

1. Device for fixing and protecting a needle, this device comprising a first and a second adhesive part (3, 8), separated by a central part (7), the first part (3) being intended to be fixed to the skin, opposite the chosen penetration site for the needle, only one of the faces of the first part (3) being adhesive, this device being **characterized in that** it comprises a cutting line (12) defining two longitudinal arms (22, 23), each of these two arms (22, 23) comprising an outermost part belonging to the second adhesive part (8).

2. Device according to Claim 1, **characterized in that** the first part (3) is adhesive on its front face, the second part (8) being adhesive on its back face only.

3. Device according to Claim 1 or 2, **characterized in that** it is made of an elastic material, in particular elastic in the longitudinal and transverse directions.

4. Device according to any one of Claims 1 to 3, **characterized in that** it is made of transparent, translucent or semi-transparent material, at least with regard to its central part.

5. Device according to any one of the preceding claims, **characterized in that** it comprises a slit (2) extending in its central part (7).

6. Device according to Claim 5, **characterized in that** the slit is longitudinal (2) and extends at least partly into the first adhesive part (3).

7. Device according to any one of the preceding claims, **characterized in that** the second part (8) is substantially identical to the first part (3) in shape, for example oval, square or rectangular.

8. Perfusion kit comprising a device as presented in any one of the preceding claims and a needle (20).
